## Europäisches Patentamt

## European Patent Office

## Office européen des brevets

(11) Publication number: **0 192 289**
**B1**

# EUROPEAN PATENT SPECIFICATION

(45) Date of publication of patent specification: **13.12.89**

(51) Int. Cl.⁴: **C 07 C 2/00, B 01 J 29/28, B 01 J 29/04**

(21) Application number: **86200138.5**

(22) Date of filing: **31.01.86**

(54) Process for the preparation of an aromatic hydrocarbons-containing mixture.

(30) Priority: **22.02.85 NL 8500501**

(43) Date of publication of application:
**27.08.86 Bulletin 86/35**

(45) Publication of the grant of the patent:
**13.12.89 Bulletin 89/50**

(84) Designated Contracting States:
**DE GB NL**

(56) References cited:
**EP-A-0 107 875**
**EP-A-0 160 335**
**CA-A-1 122 620**
**GB-A-2 021 636**
**US-A-4 227 992**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Kieffer, Eduard Philip**
**Badhuisweg 3**
**NL-1031 CM Amsterdam (NL)**
Inventor: **Sie, Swan Tiong**
**Badhuisweg 3**
**NL-1031 CM Amsterdam (NL)**

(74) Representative: **Aalbers, Onno et al**
**P.O. Box 302**
**NL-2501 CH The Hague (NL)**

**EP 0 192 289 B1**

**Description**

The invention relates to a process for the preparation of an aromatic hydrocarbons-containing mixture from wet natural gas and to aromatic hydrocarbons-containing mixtures thus obtained.

Natural gas comprising a major part of methane and ethane may also contain considerable quantities of paraffins with at least three carbon atoms (hereinafter referred to as $C_3^+$ paraffins). When it is to be transported through gas pipelines or as liquefied natural gas, such as $C_3^+$ paraffins present in this so-called "wet natural gas" should be removed. This can be accomplished step-wise, by adsorption and/or cooling, whereby first the wet gas is divided into what is called a "residual gas" ($C_1$—$C_4$) and a $C_5^+$ fraction, followed by separation of the residual gas into what is called a "dry gas" ($C_1/C_2$) and a $C_3/C_4$ fraction. A drawback to these physical separations is that they are rather costly and that they yield products which are not very desirable either for reasons of transport and storage ($C_3/C_4$) or because of their octane quality ($C_5^+$).

The present invention results from an investigation into the possibility of catalytically converting $C_3^+$ paraffins present in residual or wet natural gas containing more than 50%w of methane, into an aromatic hydrocarbons-containing mixture, instead of removing these paraffins by means of physical separation.

The conversion can be carried out by using catalysts comprising a crystalline silicate containing at least one of the metals aluminium and gallium which silicate has a special structure. The crystalline silicates referred to are characterized in that, after one hour's calcination in air at 500°C, they have the following properties:

a) an X-ray powder diffraction pattern in which the strongest lines are the lines given in Table A,

TABLE A

| d(Å) |
| --- |
| 11.1 ±0.1 |
| 10.1 ±0.1 |
| 3.84±0.07 |
| 3.72±0.06 |

b) and in the formula which represents the composition of the silicate, expressed as moles of the oxides, the $SiO_2/Al_2O_3$ molar ratio is 25—400 and/or the $SiO_2/Ga_2O_3$ molar ratio is 25—250.

As described in EP—A—107875, gallium silicates can very suitably be used for converting $C_3/C_4$ paraffins in a stable operation into aromatic gasoline of high quality (low $C_{11}^+$ aromatics content). The conversion proceeds very selectively with only a minor quantity of $C_1/C_2$ paraffins being formed as by-products. Unlike gallium silicates, the use of aluminium silicates as catalysts resulted in the formation of a considerable quantity of $C_1/C_2$ paraffins as by-products. Since it was further found that conversion of pure $C_3/C_4$ paraffins at a high pressure (>20 bar) yielded a gasoline of low quality (high $C_{11}^+$ aromatics content), whilst conversion of highly diluted $C_3/C_4$ streams (such as residual or wet natural gas) at a low pressure (<10 bar) led to rapid deactivation of the catalyst, it had to be assumed that the favourable results obtained with crystalline gallium silicates could only be achieved if the conversion was carried out at a low pressure (<10 bar) and by using a feed which consisted substantially of $C_3/C_4$ paraffins (>50%w).

It has now been found that $C_3^+$ paraffins present in natural gas with a methane content higher than 50%w, which gas is subject to a pressure higher than 20 bar, can be converted in a stable operation into aromatic gasoline of high quality by using a catalyst comprising a crystalline gallium silicate. Since when natural gas is used as feedstock the occurrence of considerable quantities of $C_1/C_2$ paraffins as by-product is no longer a drawback, the present conversion can also be carried out by using a catalyst comprising a crystalline aluminium or aluminium/gallium silicate or a physical mixture of aluminium—and/or gallium silicates.

The present invention therefore relates to a process for the preparation of an aromatic hydrocarbons-containing mixture wherein a natural gas containing more than 50%w of methane and furthermore paraffins with at least three carbon atoms per molecule is contacted at a pressure higher than 20 bar and at elevated temperature with a catalyst comprising a crystalline silicate containing at least one of the metals aluminium and gallium, which silicate has the following properties after one hour's calcination in air at 500°C:

a) an X-ray powder diffraction pattern in which the strongest lines are the lines given in Table A, and

b) in the formula which represents the composition of the silicate, expressed as moles of the oxides, the $SiO_2/Al_2O_3$ molar ratio is 25—400 and the $SiO_2/Ga_2O_3$ molar ratio is 25—250.

The complete X-ray powder diffraction pattern of a typical example of the employed crystalline metal silicates after one hour's calcination in air at 500°C is given in Table B.

2

EP 0 192 289 B1

TABLE B

| d(Å) | | Rel. int. | d(Å) | Rel. int. |
|---|---|---|---|---|
| 11.1 | | 100 | 3.84 (D) | 57 |
| 10.0 | (D) | 70 | 3.72 (D) | 31 |
| 8.93 | | 1 | 3.63 | 16 |
| 7.99 | | 1 | 3.47 | <1 |
| 7.42 | | 2 | 3.43 | 5 |
| 6.68 | | 7 | 3.34 | 2 |
| 6.35 | | 11 | 3.30 | 5 |
| 5.97 | | 17 | 3.25 | 1 |
| 5.70 | | 7 | 3.05 | 8 |
| 5.56 | | 10 | 2.98 | 11 |
| 5.35 | | 2 | 2.96 | 3 |
| 4.98 | (D) | 6 | 2.86 | 2 |
| 4.60 | | 4 | 2.73 | 2 |
| 4.35 | | 5 | 2.60 | 2 |
| 4.25 | | 7 | 2.48 | 3 |
| 4.07 | | 2 | 2.40 | 2 |
| 4.00 | | 4 | | |

(D)=doublet

In the process according to the invention it is preferred to use a crystalline silicate having a $SiO_2/Al_2O_3$ molar ratio of 25—250 and/or a $SiO_2/Ga_2O_3$ molar ratio of 60—220.

When a catalyst comprising a crystalline gallium silicate or aluminium/gallium silicate is used, said catalyst is preferably subjected once or several times to a two-step treatment, hereinafter referred to as "redox treatment", which comprises a first step in which the catalyst is contacted for at least 15 minutes, at a temperature of 350—700°C, with a hydrogen-containing reducing gas, and a second step in which the catalyst is contacted for at least 15 minutes, at a temperature of 350—700°C, with an oxygen-containing oxidizing gas. It is preferred to carry out the redox treatment not more than three times when the $SiO_2/Ga_2O_3$ molar ratio in the crystalline silicate is at most 110, and three to ten times when the molar ratio is from 110—130. When use is made of a crystalline silicate with a $SiO_2/Ga_2O_3$ molar ratio higher than 130 but not higher than 220, it is preferred to subject the silicate first to a calcination treatment at a temperature of 600—1000°C, after which the redox treatment is carried out at least three and at most ten times.

In the first step of the redox treatment the catalyst should be contacted for at least 15 minutes, at a temperature of 350—700°C with a hydrogen-containing reducing gas. In principle the first step of the redox treatment can be carried out in two ways. Firstly, the hydrogen-containing reducing gas can be supplied to the catalyst from an external source. In this case it is preferred to use a gas containing at least 20%v and in particular at least 40%v of hydrogen. In addition to hydrogen the gas used may very suitably contain either substantially nitrogen, or substantially carbon monoxide, or substantially $C_4^-$ hydrocarbons. Suitable gases which, in addition to hydrogen, contain substantially carbon monoxide can be obtained as synthesis gas, by gasification starting from a heavy carbonaceous material such as coal, or by steam reforming or partial oxidation starting from light hydrocarbons such as natural gas. Suitable gases which, in addition to hydrogen, contain substantially $C_4^-$ hydrocarbons can be obtained as by-product in the catalytic conversion of hydrocarbons in the presence of hydrogen, such as cracking isomerization and reforming. Secondly, the hydrogen-containing reducing gas can be allowed to form in situ by contacting the catalyst for at most five hours with a hydrocarbon or mixture of hydrocarbons. In this case the hydrocarbon or mixture of hydrocarbons used can very suitably be the feedstock to be used in the process.

In the second step of the redox treatment the catalyst should be contacted for at least 15 minutes, at a temperature of 350—700°C, with an oxygen-containing oxidizing gas. The gas used preferably contains at least 5%v and in particular at least 10%v of oxygen. The second step of the redox treatment can very suitably be carried out by using a gas which, in addition to oxygen, contains either substantially nitrogen, or substantially nitrogen, carbon monoxide and carbon dioxide. A suitable gas which, in addition to oxygen, contains substantially nitrogen, is air. Suitable gases which, in addition to oxygen, contain substantially nitrogen, carbon monoxide and carbon dioxide, are exhaust gases obtained in the removal of coke from deactivated hydrocarbon conversion catalysts by means of an excess of air. It is preferred to carry out the two steps of the redox treatment at a temperature of 400—650°C and in particular at a temperature of 475—575°C. Furthermore, it is preferred to carry out the two steps of the redox treatment at the same temperature.

The preparation of the crystalline metal silicates which are used in the process according to the invention can be carried out very suitably by starting from an aqueous mixture comprising the following compounds: one or more compounds of an alkali metal (M), one or more organic nitrogen compounds

3

(RN) which contain an organic cation, or from which an organic cation is formed during the preparation of the silicate, one or more silicon compounds and one or more aluminium and/or gallium compounds. The preparation is carried out by maintaining the mixture at an elevated temperature until the silicate has formed, subsequently separating silicate crystals from the mother liquor and washing, drying and calcining the crystals. In the aqueous mixture from which the silicates are prepared the various compounds should be present in the following molar ratios, expressed—with the exception of the organic nitrogen compounds—as moles of the oxides:

$M_2O:SiO_2=0.01$—0.35,

$RN:SiO_2=0.02$—1.0,

$SiO_2:Al_2O_3=25$—1000, and/or $SiO_2:Ga_2O_3=25$—400, and

$H_2O:SiO_2=5$—65.

In the preparation of the silicates the starting material may very suitably be a mixture in which a quaternary ammonium compound is present as organic nitrogen compound, a sodium compound as alkali metal compound and amorphous silica as silicon compound.

The silicates prepared as described hereinbefore contain alkali metal ions. By using suitable exchange methods these can be replaced by other cations, such as hydrogen ions or ammonium ions. The crystalline metal silicates which are used in the process according to the invention preferably have an alkali metal content of less than 0.05%w. In the process according to the invention the crystalline metal silicates can be used either alone or in combination with a binder material, such as refractory oxides, kaoline or bentonite.

The process according to the invention is preferably carried out at a temperature of 400—700°C and in particular of 500—600°C, a pressure of 20—150 bar and in particular of 20—100 bar and a space velocity of 0.1—10 kg $C_3^+ \cdot kg^{-1} \cdot h^{-1}$ and in particular of 0.2—5 kg $C_3^+ \cdot kg^{-1} \cdot h^{-1}$.

The process according to the invention can suitably be employed as an independent process in which, in addition to a dry natural gas, an aromatic gasoline is obtained as product. The process according to the invention is of particular importance in that it can be combined with a recently developed process for the preparation of middle distillates from natural gas. In this process natural gas is converted into synthesis gas and subsequently the synthesis gas is converted into a paraffinic hydrocarbon mixture in the presence of a special cobalt-containing catalyst. Finally, at least the high-boiling fraction of the product prepared with said catalyst is subjected to a hydrocracking treatment in the presence of a catalyst comprising one or more noble metals from Group VIII supported on a carrier. The particular property of the special cobalt-containing catalysts used in this process is that they yield a product substantially consisting of unbranched paraffins of which a high-boiling fraction can be converted in high yield into middle distillates by means of hydrocracking. The cobalt-containing catalysts involved comprise silica, alumina or silica-alumina as a carrier and cobalt together with zirconium, titanium, chromium and/or ruthenium as catalytically active metals and have preferably been prepared by applying the metals involved to the carrier material by impregnation and/or kneading. In the process for the preparation of middle distillates described hereinbefore the starting material may be wet, residual or dry natural gas, at choice. However, if the natural gas available contains $C_3^+$ paraffins, it should preferably first be subjected to the process according to the invention, and for the following reason. Both in the conversion over the cobalt-containing catalyst and in the hydrocracking treatment a low-value paraffinic gasoline is formed as by-product. The process according to the invention yields a high-value aromatic gasoline. By mixing the aromatic gasoline (i.e. the aromatic hydrocarbons-containing mixture) obtained in the process according to the invention with paraffinic gasoline (i.e. a paraffinic hydrocarbons-containing mixture) obtained in the middle distillate synthesis, a gasoline can be composed which has an excellent quality.

In the middle distillate synthesis described hereinbefore the conversion of the natural gas into synthesis gas is preferably carried out by steam reforming or partial oxidation. The conversion of the synthesis gas into a paraffinic hydrocarbon mixture is preferably carried out at a temperature of 175—275°C and a pressure of 10—75 bar in the presence of a catalyst comprising cobalt and zirconium on silica as carrier. The hydrocracking treatment is preferably carried out at a temperature of 250—350°C and a pressure of 10—75 bar in the presence of a catalyst comprising 0.2—1%w platinum or palladium on silica-alumina as carrier.

The invention is illustrated with the aid of the following Example.

Example

A crystalline gallium silicate (Silicate 1) was prepared by heating a mixture comprising NaOH, amorphous silica, $(C_3H_7)_4NOH$ and $Ga(NO_3)_3$ in water for 24 hours in an autoclave under autogenous pressure at 150°C. After cooling of the reaction mixture the silicate formed was filtered off, washed with water until the pH of the wash water was about 8, and dried at 120°C. After one hour's calcination in air at 500°C Silicate 1 had the following properties:

a) an X-ray powder diffraction pattern substantially corresponding with that mentioned in Table B, and

b) a $SiO_2/Ga_2O_3$ molar ratio of 70.

Silicate 1A was prepared by boiling Silicate 1 with a 1.0 molar $NH_4NO_3$ solution, washing, drying at 120°C and calcining at 500°C.

Silicate 1A was subjected three times to a redox treatment comprising a first step in which the silicate was contacted for 30 minutes, at a temperature of 550°C and a pressure of 1.5 bar, with an $H_2/N_2$ mixture

having a 1:1 volume ratio, followed by a second step in which the silicate was contacted for 1 hour, at a temperature of 550°C and a pressure of 1.5 bar, with air. Thus Silicate 1B was obtained from Silicate 1A.

Silicate 1B was tested in four experiments (Experiments 1—4) as catalyst employed in the preparation of an aromatic hydrocarbon mixture starting from n-butane and wet natural gas. The experiments were carried out in a reactor containing a fixed catalyst bed. The conditions under which the experiments were carried out and the results are given in Table C.

Of the experiments mentioned in Table C, only Experiment 4 is an experiment according to the invention. Comparative experiments 1—3 fall outside the scope of the invention. They have been included for comparison. Experiment 4 was carried out at a pressure higher than 20 bar using natural gas containing $C_3^+$ paraffins and having a methane content of over 50%w. In this experiment a high-quality gasoline was obtained in a stable operation. Experiments 1 and 2 were carried out using n-butane as feedstock. Experiment 2 was carried out at a pressure higher than 20 bar but it yielded a low-quality gasoline. In Experiment 3 a suitable natural gas was used as feedstock, but it was carried out at a pressure below 20 bar. This experiment led to very rapid catalyst deactivation.

TABLE C

Experiments carried out using n-butane as feed, at 550°C and a space velocity of $2 \, \text{kg} \cdot \text{kg}^{-1} \cdot \text{h}^{-1}$

Experiment 1 at 6 bar
At run hour 2   : $C_5^+$ yield 42.7%w with a $C_{11}^+$ aromatics content 8.9% *)
At run hour 100: $C_5^+$ yield 41.9%w with a $C_{11}^+$ aromatics content 9.4%

Experiment 2 at 40 bar
At run hour 2   : $C_5^+$ yield 39.9%w with a $C_{11}^+$ aromatics content 18.5%

Experiments carried out using wet natural gas, at 550°C and a space velocity of $1 \, \text{kg} \, C_3^+ \cdot \text{kg}^{-1} \cdot \text{h}^{-1}$
(Composition of the natural gas, in %w: $C_1$ 58.6; $C_2$ 13.6; $C_3$ 14.4; $C_4$ 9.2; $C_5^+$ 1.7; $CO_2$ 1.5; $N_2$ 1.0)

Experiment 3 at 6 bar
At run hour 2   : $C_5^+$ yield (calculated on $C_3^+$ in the feed) 46.7%w with a $C_{11}^+$ aromatics content of 10.5%
At run hour 100: $C_5^+$ yield 15.1%w with a $C_{11}^+$ aromatics content 9.2%

Experiment 4 at 40. bar
At run hour 2   : $C_5^+$ yield 49.2%w with a $C_{11}^+$ aromatics content 9.8%
At run hour 100: $C_5^+$ yield 46.2%w with a $C_{11}^+$ aromatics content 9.7%

$$\text{*) } C_{11}^+ \text{ aromatics content} = \frac{\text{\%w } C_{11}^+ \text{ aromatics}}{\text{\%w } C_6^+ \text{ aromatics}} \times 100$$

**Claims**

1. A process for the preparation of an aromatic hydrocarbons-containing mixture wherein a feedstock comprising paraffins with at least three carbon atoms per molecule is contacted at an elevated temperature with a catalyst comprising a crystalline silicate containing at least one of the metals aluminium and gallium, which silicate, after one hour's calcination in air at 500°C, has an X-ray powder diffraction pattern in which the strongest lines are the lines given in Table A, characterized in that the feedstock is a natural gas containing more than 50%w of methane together with said paraffins with at least three carbon atoms per molecule, the contacting is carried out at a pressure higher than 20 bar and, in the formula which represents the composition of the silicate, expressed as moles of the oxides, the $SiO_2/Al_2O_3$ molar ratio is 25—400 and/or the $SiO_2/Ga_2O_3$ molar ratio is 25—250.

2. A process as claimed in claim 1 wherein the crystalline silicate has a $SiO_2/Al_2O_3$ molar ratio of 25—250 and/or a $SiO_2/Ga_2O_3$ molar ratio of 60—220.

3. A process as claimed in claim 1 or 2 wherein the catalyst comprises a crystalline gallium silicate or an aluminium/gallium silicate which catalyst has been subjected at least once to a two-step redox treatment comprising a first step in which the catalyst is contacted for at least 15 minutes and at a temperature of 350—700°C with a hydrogen-containing reducing gas and a second step in which the catalyst is contacted for at least 15 minutes and at 350—700°C with an oxygen-containing oxidizing gas.

4. A process as claimed in claim 3 wherein the crystalline silicate has a $SiO_2/Ga_2O_3$ molar ratio of at most 130 and the redox treatment is carried out not more than three times when the molar ratio is at most 110 and three to ten times when the molar ratio is from 110—130.

5. A process as claimed in claim 3 or 4, wherein the first step of the redox treatment is carried out using a hydrogen-containing reducing gas which is formed in situ by contacting the catalyst for at most five hours with hydrocarbons.

6. A process as claimed in any of claims 3—5 wherein the two steps of the redox treatment are carried out at a temperature from 400—650°C, and preferably from 475—575°C.

7. A process as claimed in any of the preceding claims wherein the crystalline metal silicates have been prepared by maintaining an aqueous mixture comprising the following compounds: one or more compounds of an alkali metal (M), one or more organic nitrogen compounds (RN) which contain an organic cation, or from which an organic cation is formed during the preparation of the silicate, one or more silicon compounds and one or more aluminium and/or gallium compounds, in which mixture the various compounds are present in the following molar ratios:

$M_2O:SiO_2=0.01—0.35$,
$RN:SiO_2=0.02—1.0$,
$SiO_2:Al_2O_3=25—1000$, and/or $SiO_2:Ga_2O_3=25—400$, and
$H_2O:SiO_2=5—65$,

at an elevated temperature until the silicate has formed, subsequently separating silicate crystals from the mother liquor and calcination.

8. A process as claimed in claim 7 wherein the aqueous mixture contains a quaternary ammonium compound as organic nitrogen compound, a sodium compound as alkali metal compound and amorphous silica as silicon compound.

9. A process as claimed in any of the preceding claims which is carried out at a temperature of 400—700°C, a pressure of 20—150 bar and a space velocity of $0.1—10 \, kg \, C_3^+ \cdot kg^{-1} \cdot h^{-1}$.

10. A process for the preparation of middle distillates and gasoline which comprises as first step a process as claimed in any one of claims 1—9 in which a natural gas comprising $C_3^+$ paraffins is converted into a dry natural gas and an aromatic hydrocarbons-containing mixture, which dry natural gas is converted in a second step into synthesis gas which is converted in a third step into a paraffinic hydrocarbons-containing mixture in the presence of a catalyst comprising silica, alumina, or silica-alumina as carrier and cobalt together with zirconium, titanium, chromium and/or ruthenium as catalytically active metals, at least the high-boiling fraction of the product obtained from the third step is subjected in a fourth step to a hydrocracking treatment in the presence of a catalyst comprising one or more noble metals from Group VIII supported on a carrier, and a gasoline is prepared by mixing at least part of the aromatic hydrocarbons-containing mixture obtained in the first step with at least part of the paraffinic hydrocarbons-containing mixture obtained in the third step and/or in the fourth step.

11. A process as claimed in claim 10, wherein the second step is carried out by steam reforming or partial oxidation, the third step is carried out at a temperature of 175—275°C and a pressure of 10—75 bar in the presence of a catalyst comprising cobalt and zirconium on silica as carrier, and the fourth step is carried out at a temperature of 250—350°C and a pressure of 10—75 bar in the presence of a catalyst comprising 0.2—1%w of platinum or palladium on silica-alumina as carrier.

**Patentansprüche**

1. Ein Verfahren zur Herstellung einer aromatische Kohlenwasserstoffe enthaltenden Mischung, in welchem eine Paraffine mit mindestens 3 Kohlenstoffatomen je Molekül enthaltende Zuspeisung bei erhöhter Temperatur mit einem Katalysator kontaktiert wird, welcher ein kristallines Silikat umfaßt, das mindestens eines der Metalle Aluminium und Gallium enthält, und welches Silikat nach einstündiger Calcinierung in Luft bei 500°C ein Röntgenbeugungs - Pulverdiagramm aufweist, dessen stärkste Linien die in Tabelle A angegebenen Linien sind, dadurch gekennzeichnet, daß die Zuspeisung ein mehr als 50 Gew.% Methan zusammen mit besagten Paraffinen mit mindestens 3 Kohlenstoffatomen pro Molekül enthaltendes Erdgas ist, daß das Kontaktieren bei einem Druck von mehr als 20 bar durchgeführt wird und daß in der die Zusammensetzung des Silikats wiedergebenden Formel, ausgedrückt als Mole der Oxide, das Molverhältnis $SiO_2:Al_2O_3$ 25 bis 400 und/oder das Molverhältnis $SiO_2:Ga_2O_3$ 25 bis 250 beträgt.

2. Ein Verfahren wie in Anspruch 1 beansprucht, in welchem das kristalline Silikat ein Molverhältnis $SiO_2:Al_2O_3$ von 25 bis 250 und/oder ein Molverhältnis $SiO_2:Ga_2O_3$ von 60 bis 220 hat.

3. Ein Verfahren wie in einem der Ansprüche 1 oder 2 beansprucht, in welchem der Katalysator ein kristallines Galliumsilikat oder ein Aluminium - Gallium - Silikat umfaßt, welcher Katalysator mindestens ein Mal einer zweistufigen Redox-Behandlung unterworfen worden ist, welche eine erste Stufe, in welcher der Katalysator mindestens 15 Minuten lang und bei einer Temperatur von 350 bis 700°C mit einem wasserstoffhaltigen reduzierenden Gas kontaktiert wird, und eine zweite Stufe umfaßt, in welcher der Katalysator mindestens 15 Minuten lang und bei 350 bis 700°C mit einem sauerstoffhaltigen oxidierenden Gas kontaktiert wird.

4. Ein Verfahren wie in Anspruch 3 beansprucht, in welchem das kristalline Silikat ein Molverhältnis $SiO_2:Ga_2O_3$ von höchstens 130 aufweist und die Redox-Behandlung nicht mehr als 3 Mal durchgeführt wird, falls das Molverhältnis einen Wert von höchstens 110 hat, und daß sie drei bis zehn Mal durchgeführt wird, wenn das Molverhältnis einen Wert von 110 bis 130 hat.

5. Ein Verfahren wie in Anspruch 3 oder 4 beansprucht, in welchem die erste Stufe der Redox-Behandlung unter Verwendung eines wasserstoffhaltigen reduzierenden Gases durchgeführt wird, welches in situ durch Kontaktieren des Katalysators während höchstens 5 Stunden mit Kohlenwasserstoffen gebildet worden ist.

6

6. Ein Verfahren wie in irgendeinem der Ansprüche 3 bis 5 beansprucht, in welchem die beiden Stufen der Redox-Behandlung bei einer Temperatur von 400 bis 650°C und vorzugsweise von 475 bis 575°C durchgeführt werden.

7. Ein Verfahren wie in irgendeinem der vorhergehenden Ansprüche beansprucht, in welchem die kristallinen Metallsilikate hergestellt worden sind durch Halten einer wässrigen Mischung, welche die nachstehend aufgeführten Verbindungen enthält, auf einer erhöhten Temperatur, bis sich das Silikat gebildet hat, anschließendes Abtrennen der Silikatkristalle aus der Mutterlauge und Calcinierung: eine oder mehrere Verbindungen eines Alkalimetalls (M), eine oder mehrere organische Stickstoffverbindungen (RN), welche ein organisches Kation enthalten oder aus denen sich während der Herstellung des Silikats ein organisches Kation bildet, ein oder mehrere Siliciumverbindungen und ein oder mehrere Aluminium- und/oder Galliumverbindungen, in welcher Mischung die verschiedenen Verbindungen in den folgenden molaren Verhältnissen vorliegen:

$M_2O:SiO_2=0,01—0,35,$
$RN:SiO_2=0,02—1,0,$
$SiO_2:Al_2O_3=25—1000$ und/oder $SiO_2:Ga_2O_3=25—400,$ und
$H_2O:SiO_2=5—65.$

8. Ein Verfahren, wie in Anspruch 7 beansprucht, in welchem die wässrige Mischung eine quaternäre Ammoniumverbindung als organische Stickstoffverbindung, eine Natriumverbindung als Alkalimetallverbindung und amorphe Kieselsäure als Siliciumverbindung enthält.

9. Ein Verfahren wie in irgendeinem der vorhergehenden Ansprüche beansprucht, welches bei einer Temperatur von 400 bis 700°C, einem Druck von 20 bis 150 bar und einer Raumgeschwindigkeit von 0,1 bis 10 kg $C_3^+ \cdot kg^{-1} \cdot h^{-1}$ durchgeführt wird.

10. Ein Verfahren zur Herstellung von Mitteldestillaten und Benzin, welches als erste Stufe ein Verfahren umfaßt, wie in irgendeinem der Ansprüche 1 bis 9 beansprucht, in welcher ein $C_3^+$-Paraffine enthaltendes Erdgas in ein trockenes Erdgas und eine aromatische Kohlenwasserstoffe enthaltende Mischung umgewandelt wird, welches trockene Erdgas in einer zweiten Stufe in Synthesegas umgewandelt wird, welches wiederum in einer dritten Stufe in Anwesenheit eines Katalysators, welcher $SiO_2$, $Al_2O_3$ oder $SiO_2—Al_2O_3$ als Trägermaterial und Kobalt zusammen mit Zirkonium, Titan, Chrom und/oder Ruthenium als katalytisch aktive Metalle umfaßt, in eine paraffinische Kohlenwasserstoffe enthaltende Mischung umgewandelt wird, in welchem mindestens die hochsiedende Fraktion des aus der dritten Stufe erhaltenen Produktes in einer vierten Stufe einer Hydrocrackbehandlung in Anwesenheit eines Katalysators unterworfen wird, der ein oder mehrere Edelmetalle aus der Gruppe VIII auf einem Trägermaterial enthält, und in welchem ein Benzin durch Vermischen mindestens eines Teils der aus der ersten Stufe erhaltenen, aromatische Kohlenwasserstoffe enthaltenden Mischung mit mindestens einem Teil der in der dritten und/oder vierten Stufe erhaltenen paraffinische Kohlenwasserstoffe enthaltenden Mischung hergestellt wird.

11. Ein Verfharen wie in Anspruch 10 beansprucht, in welchem die zweite Stufe durch Dampfreformierung oder Teiloxidation, die dritte Stufe bei einer Temperatur von 175 bis 275°C und einem Druck von 10 bis 75 bar in Anwesenheit eines Katalysators, welcher Kobalt und Zirkonium auf Siliciumdioxid als Trägermaterial enthält, und die vierte Stufe bei einer Temperatur von 250 bis 350°C und einem Druck von 10 bis 75 bar in Anwesenheit eines Katalysators, welcher 0,2 bis 1 Gew.% Platin oder Palladium auf einem Siliciumdioxid - Aluminiumoxid als Träger enthält, durchgeführt wird.

## Revendications

1. Procédé de préparation d'un mélange contenant des hydrocarbures aromatiques, selon lequel on met en contact à une température élevée une charge comprenant des paraffines qui contiennent au moins 3 atomes de carbone par molécule avec un catalyseur comprenant un silicate cristallin contenant au moins l'un des métaux aluminium et gallium, lequel silicate, après une heure de calcination à l'air à 500°C, présente en poudre un motif de diffraction par rayons X dont les lignes les plus fortes sont les lignes indiquées dans le tableau A, caractérisé en ce que la charge est un gaz naturel contenant plus de 50% en poids de méthane conjointement avec lesdites paraffines contenant au moins trois atomes de carbone par molécule, on effectue la mise en contact sous une pression supérieure à 20 bars et dans la formule qui représente la composition du silicate exprimée en moles des oxydes, le rapport molaire $SiO_2/Al_2O_3$ est de 25 à 400 et/ou le rapport molaire $SiO_2/Ga_2O_3$ est de 25 à 250.

2. Procédé selon la revendication 1, dans lequel le silicate cristallin présente un rapport molaire $SiO_2/Al_2O_3$ de 25 à 250 et/ou un rapport molaire $SiO_2/Ga_2O_3$ de 60 à 220.

3. Procédé selon la revendication 1 ou 2, dans lequel le catalyseur comprend un silicate de gallium cristallin ou un silicate d'aluminium/gallium, lequel catalyseur a été soumis une fois au moins à un traitement redox en deux étapes comprenant un premier stade dans lequel on met en contact le catalyseur pendant 15 minutes au moins et à une température de 350 à 700°C avec un gaz réducteur contenant de l'hydrogène et un second stade dans lequel on met le catalyseur en contact pendant 15 minutes au moins et à une température de 350 à 700°C avec un gaz oxydant contenant de l'oxygène.

4. Procédé selon la revendication 3, dans lequel le silicate cristallin présente un rapport molaire

$SiO_2/Ga_2O_3$ qui ne dépasse pas 130 et on effectue le traitement redox pas plus de trois fois quand le rapport molaire ne dépasse pas 110 et de trois à dix fois quand le rapport molaire est de 110 à 130.

5. Procédé selon la revendication 3 ou 4, dans lequel on effectue le premier stade du traitement redox en utilisant un gaz réducteur contenant de l'hydrogène que l'on forme in situ en mettant le catalyseur en contact pendant un maximum de 5 heures avec les hydrocarbures.

6. Procédé selon l'une quelconque des revendications 3 à 5, dans lequel on effectue les deux stades du traitement redox à une température de 400 à 650°C et, de préférence, entre 475 et 575°C.

7. Procédé selon l'une quelconque des revendication précédentes, dans lequel on prépare les silicates de métaux cristallins en maintenant à une température élevée un mélange aqueux qui comprend les composés suivants: un ou plusieurs composé(s) d'un métal alcalin (M), un ou plusieurs composé(s) organique(s) d'azote (RN) qui contienent un cation organique ou à partir du(des) quel(s) le cation organique a été formé pendant la préparation du silicate, un ou plusieurs composé(s) de silicium et un ou plusieurs composé(s) d'aluminium et/ou de gallium, mélange dans lequel les divers composés sont présents dans les rapports molaires suivants:

$M_2O:SiO_2$=0,01 à 0,35,
$RN:SiO_2$=0,02—1,0,
$SiO_2:Al_2O_3$=25 à 1000 et/ou $SiO_2:Ga_2O_3$=25 à 400, et
$H_2O:SiO_2$=5 à 65,

jusqu'à formation du silicate, puis on sépare les cristaux de silicate de la liqueur mère et on calcine.

8. Procédé selon la revendication 7, dans lequel le mélange aqueux contient un composé d'ammonium quaternaire à titre de composé organique azoté, un composé de sodium à titre de composé de métal alcalin et une silice amorphe à titre de composé de silicium.

9. Procédé selon l'une quelconque des revendications précédentes, qu'on effectue à une température de 400 à 700°C, une pression de 20 à 150 bars et une vitesse spatiale de 0,1 à 10 kg $C_3^+ \cdot kg^{-1} \cdot h^{-1}$.

10. Procédé de préparation de distillats médians et d'essence qui consiste à titre de la première étape en un procédé selon l'une quelconque des revendications 1 à 9, dans lequel on convertit un gaz naturel comprenant des paraffines en $C_3^+$ en un gaz naturel sec et un mélange aromatique contenant des hydrocarbures aromatiques, ce gaz naturel sec étant converti dans une seconde étape en un gaz de synthèse qu'on convertit dans une troisième étape en un mélange contenant des hydrocarbures paraffiniques en présence d'un catalyseur comprenant de la silice, de l'alumine ou une silice-alumine en qualité de support et du cobalt conjointement avec du zirconium, du titane, du chrome et/ou du rhuténium en qualité de métaux catalytiquement actifs, on soumet au moins la fraction à haut point d'ébullition du produit.obtenu à la troisième étape au cours d'une quatrième étape à un traitement d'hydrocraquage en présence d'un catalyseur comprenant un ou plusieurs métaux nobles du Groupe VIII sur un support, et on prépare une essence en mélangeant une partie au moins du mélange contenant les hydrocarbures aromatiques provenant de la première étape avec une partie au moins du mélange contenant les hydrocarbures paraffiniques qu'on obtient à la troisième étape et/ou à la quatrième étape.

11. Procédé selon la revendication 10, dans lequel on effectue la seconde étape par reforming à la vapeur ou oxydation partielle, on effectue la troisième étape à une température de 175 à 275°C et une pression de 10 à 75 bars en présence d'un catalyseur comprenant du cobalt et du zirconium sur un support de silice, et on effectue la quatrième étape à une température de 250 à 350°C sous une pression de 10 à 75 bars en présence d'un catalyseur comprenant 0,2 à 1% en poids de platine ou de palladium sur un support de silice-alumine.